Europäisches Patentamt

European Patent Office

Office européen des brevets

(19)

(11) Veröffentlichungsnummer : **0 190 269**
**B1**

(12) **EUROPÄISCHE PATENTSCHRIFT**

(45) Veröffentlichungstag der Patentschrift :
06.07.88

(51) Int. Cl.⁴ : **A 61 M   5/20**

(21) Anmeldenummer : 85903982.8

(22) Anmeldetag : 01.08.85

(86) Internationale Anmeldenummer :
PCT/DE 85/00259

(87) Internationale Veröffentlichungsnummer :
WO/8601117 (27.02.86 Gazette 86/05)

(54) SPRITZENPUMPE.

(30) Priorität : 03.08.84 DE 3428655

(43) Veröffentlichungstag der Anmeldung :
13.08.86 Patentblatt 86/33

(45) Bekanntmachung des Hinweises auf die Patenterteilung : 06.07.88 Patentblatt 88/27

(84) Benannte Vertragsstaaten :
DE FR GB IT

(56) Entgegenhaltungen :
DE-B- 1 491 667
DE-C-   277 744
DE-C-   866 988
FR-A-   572 400
FR-A- 1 465 653
FR-A- 2 390 175
GB-A-   621 208
GB-A- 1 168 263
GB-A- 1 178 738
US-A- 2 702 547
US-A- 2 786 468
US-A- 3 313 291
US-A- 3 858 581

(73) Patentinhaber : Fresenius AG
Gluckensteinweg 5
D-6380 Bad Homburg v.d.H. (DE)

(72) Erfinder : FAESER, Ulrich
Brunnenweg 2
D-6242 Kronberg 2 (DE)
Erfinder : MAHN, Dieter
An den Brunnengärten 6
D-6380 Bad Homburg (DE)

(74) Vertreter : Luderschmidt, Wolfgang, Dr. Dipl-Chem. et
al
Görtz, Dr. Fuchs, Dr. Luderschmidt Patentanwälte
Sonnenberger Strasse 100 Postfach 26 26
D-6200 Wiesbaden (DE)

## Beschreibung

Die Erfindung betrifft eine Spritzenpumpe gemäß dem Oberbegriff des Anspruchs 1.

Spritzenpumpen werden im klinischen Bereich und in der medizinischen Forschung verwendet, um dem Patienten kleinere Flüssigkeitsmengen über einen längeren Zeitraum zu applizieren.

Eine dem Oberbegriff des Anspruchs 1 entsprechende Spritzenpumpe ist beispielsweise aus der US-PS 4,191,187 bekannt.

Aus dieser Druckschrift ist eine Spritzenpumpe zur dosierenden Förderung einer Flüssigkeit aus einem Spritzenzylinder mittels eines Spritzenkolbens wenigstens einer auswechselbaren Spritze bekannt. Diese bekannte Spritzenpumpe weist ein Gehäuse mit einer Halterung für die Spritze und einen motorischen Antrieb mit einem Schraubspindelgetriebe zur Übertragung der motorischen Antriebskraft vom Antrieb auf die Spritze auf. Hierbei weist das Schraubspindelgetriebe ein axial verschiebbares Antriebselement auf, dessen eines Ende mit dem beweglichen Spritzenkolben außerhalb des Gehäuses in Eingriff bringbar ist, um das Spritzenteil translatorisch zu bewegen. Ferner ist eine in einem Lagerbock angeordnete translatorisch bewegbare Kupplung vorgesehen, die ein mit einem Gewindesegment versehenes Ausrückglied aufweist, um eine Antriebsverbindung zwischen dem Antriebselement und dem Spritzenteil herstellen zu können. Weiterhin trägt der Lagerbock ein längliches Führungsstück, das im Gleitsitz auf der Gewindespindel angeordnet ist, wobei das Ausrückglied der Kupplung am Führungsstück und/oder am Lagerbock verschiebbar gelagert ist und wobei ferner das Ausrückglied ein quer zur Gewindespindel verschiebbar gelagerter Kupplungsbolzen ist, der eine sich quer zu seiner Längsachse erstreckende, im wesentlichen U-förmige Ausnehmung aufweist, durch die sich die Gewindespindel mit zumindest einem Teil ihres Umfanges erstreckt. Hierbei ist an einer Flanke der U-förmigen Ausnehmung das Gewindesegment vorgesehen.

Die gattungsgemäße Spritzenpumpe weist jedoch den hauptsächlichen Nachteil auf, daß die Gewindespindel über ihre gesamte Länge außerhalb des Gehäuses angeordnet ist. Dies ergibt das Problem, daß die Gewindespindel im Betrieb sehr leicht verschmutzt, was zu der Gefahr führt, daß die für eine genaue Dosierung erforderliche Leichtgängigkeit des Führungsstückes bzw. des mit diesem verbundenen Antriebselementes verschlechtert wird, wobei sogar die Gefahr besteht, daß beim Auftreten starker Verschmutzungen eine Bewegung vollständig unmöglich wird. Dies wiederum führt zu dem Problem, daß die gattungsgemäße Spritzenpumpe insbesondere beim Langzeitbetrieb häufig auf ihre Funktionstüchtigkeit hin überprüft werden muß, was ihre Brauchbarkeit im praktischen Betrieb stark einschränkt.

Es ist daher Aufgabe der Erfindung, eine Spritzenpumpe der im Oberbegriff des Anspruches 1 umrissenen Gattung zu schaffen, bei der die Leichtgängigkeit des Getriebes und die Funktionssicherheit auch im Langzeitbetrieb in jedem Falle gewährleistet ist.

Diese Aufgabe wird durch die kennzeichnenden Merkmale des Anspruches 1 gelöst.

Dadurch wird zunächst erreicht, daß die Gewindespindel über ihre gesamte Länge im Gehäuse angeordnet und gelagert werden kann, so daß keine Gefahr besteht, daß die Gewindespindel in irgendeiner Art und Weise verschmutzt wird, was sonst zu den zuvor anhand des gattungsgemäßen Standes der Technik erläuterten Problemen führen könnte.

Durch die Anordnung des Antriebselementes und dessen drehbewegliche Lagerung im Gehäuse wird ferner erreicht, daß die Betätigung der Kupplung bei Beibehaltung deren einfachen Aufbaues von außen möglich ist, so daß die Herstellung bzw. Trennung der Antriebsverbindung auf ebenso einfache Art und Weise wie beim gattungsgemäßen Stand der Technik möglich ist.

Zwar ist das Antriebselement der erfindungsgemäßen Spritzenpumpe bei ausgefahrerem Spritzenkolben zeit- und teilweise aus dem Gehäuse herausgeführt, jedoch treten hierbei keine mit dem gattungsgemäßen Stand der Technik vergleichbaren Probleme hinsichtlich Verschmutzung auf, da zum einen die Oberfläche des Antriebselementes sehr glattflächig ausgebildet werden kann und es zum anderen nur zu einem Teil außerhalb des Gehäuses angeordnet werden muß. Wird die erfindungsgemäße Spritzenpumpe nicht betrieben, kann das Antriebselement darüberhinaus gänzlich im Gehäuse angeordnet werden, was beim gattungsgemäßen Stand der Technik bezüglich dessen Gewindespindel nicht möglich ist, da diese ständig und in ihrer gesamten Länge außerhalb des Gehäuses angeordnet ist.

Zwar ist aus der WO 82/01998 eine Spritzenpumpe bekannt, deren Gewindespindel auch innerhalb des Gehäuses angeordnet ist, jedoch ist diese Gewindespindel an einem Ende außerhalb des Gehäuses gelagert, wozu eine mit der Gewindespindel einstückig verbundene Führungsstange vorgesehen ist, die über ihre gesamte Länge ständig außerhalb des Gehäuses liegt. Auf dieser Führungsstange wiederum ist das Antriebselement dieser Spritzenpumpe gleitbeweglich gelagert, so daß der Vorteil der Anordnung der Gewindespindel innerhalb des Gehäuses zumindest zu einem beträchtlichen Teil wieder zunichte gemacht wird. Der Grund hierfür ist, daß diese bekannte Spritzenpumpe zwei zusätzliche Dichtungen benötigt, nämlich eine zwischen der Führungsstange und dem Antriebselement und die zweite zwischen dem Antriebselement und dem Gehäuse. Dadurch wird die Leichtgängigkeit in erheblichem Maße herabgesetzt, da zur Aufrechterhaltung einer einwandfreien Funktion einer derartigen Spritzenpumpe Doppeldichtungen mit einem Abstreifring und einem Dichtring verwendet werden, die den Betrieb der Spritzenpum-

2

pe erheblich erschweren. Darüberhinaus sind die Anforderungen an die Passgenauigkeit dieser Spritzenpumpe sehr hoch, da sie an den zuvorgenannten Stellen zwischen Führungsstange, Antriebselement und Gehäuse Mehrfachführungen aufweist, bei deren Passungenauigkeit es leicht zu Verkantungen kommen kann. Genau dies wird bei der erfindungsgemäßen Spritzenpumpe vermieden, da diese lediglich eine Führung für das Antriebselement benötigt, so daß auch bei niedrigeren Anforderungen an die Passgenauigkeit Verkantungen nicht auftreten können. Darüberhinaus ist die Kupplung der aus der WO 82/01998 bekannten Spritzenpumpe gänzlich anders aufgebaut als diejenige der erfindungsgemäßen Spritzenpumpe, so daß keine vergleichbaren Verhältnisse vorliegen.

Aus der US-PS 2,702,547, Ausführungsform gemäß dortiger Fig. 1, ist es zwar auch bekannt, die Gewindespindel innerhalb des Gehäuses anzuordnen, wobei diese Spritzenpumpe eine dem gattungsgemäßen Stand der Technik sehr ähnliche Kupplung aufweist, jedoch ist zur Betätigung der Kupplung und zur Gewährleistung der Beweglichkeit des Antriebselementes eine langgestreckte Ausnehmung im Gehäuse vorgesehen, so daß letztendlich die Gewindestange auch bei dieser bekannten Spritzenpumpe stark verschmutzen kann, was zu den gleichen Problemen wie bei der gattungsgemäßen Spritzenpumpe führt.

Die Unteransprüche haben vorteilhafte Weiterbildungen der Erfindung zum Inhalt.

Zur übersetzten Umwandlung der Drehbewegung des Antriebselementes in eine translatorische Bewegung des Ausrückgliedes ist gemäß einer weiteren Ausgestaltung der Erfindung ein Steuerelement vorgesehen, das zwischen dem Antriebselement und dem Ausrückglied angeordnet ist. Dieses Steuerelement weist die Form einer drehbar gelagerten Scheibe auf, die zweckmäßig auf dem Antriebselement angeordnet und darauf befestigt ist, und die einen Mitnehmer aufweist, der mit dem Ausrückglied in Eingriff ist. Als Mitnehmer ist in baulich einfacher Weise ein exzentrisch zur Drehachse der Scheibe angeordneter Stift vorgesehen, der in eine entsprechende Ausnehmung des Ausrückgliedes eingreift. Je nachdem, wie weit der Stift von der Drehachse angeordnet ist, kann eine entsprechende Übersetzung bei der Umwandlung der Drehbewegung in eine translatorische Bewegung erreicht werden.

Weitere Einzelheiten, Merkmale und Vorteile der Erfindung ergeben sich aus der nachfolgenden Beschreibung von Ausführungsformen der Erfindung und ihrer Ausgestaltungen anhand der Zeichnung.

Es zeigen :

Fig. 1 eine Draufsicht auf eine Spritzenpumpe ;

Fig. 2 eine Ansicht der Spritzenpumpe gemäß Fig. 1 von unten ;

Fig. 3 eine Seitenansicht eines Lagerbockes ;

Fig. 4 ein als Kupplungsbolzen ausgebildetes Ausrückglied, welches, wie in Fig. 1 zu sehen ist, in den Bohrungen der Schenkel des Lagerbockes angeordnet ist,

Fig. 5 eine Seitenansicht eines Steuerelementes ;

Fig. 6 eine Vorderansicht des Steuerelementes gem. Fig. 5 ;

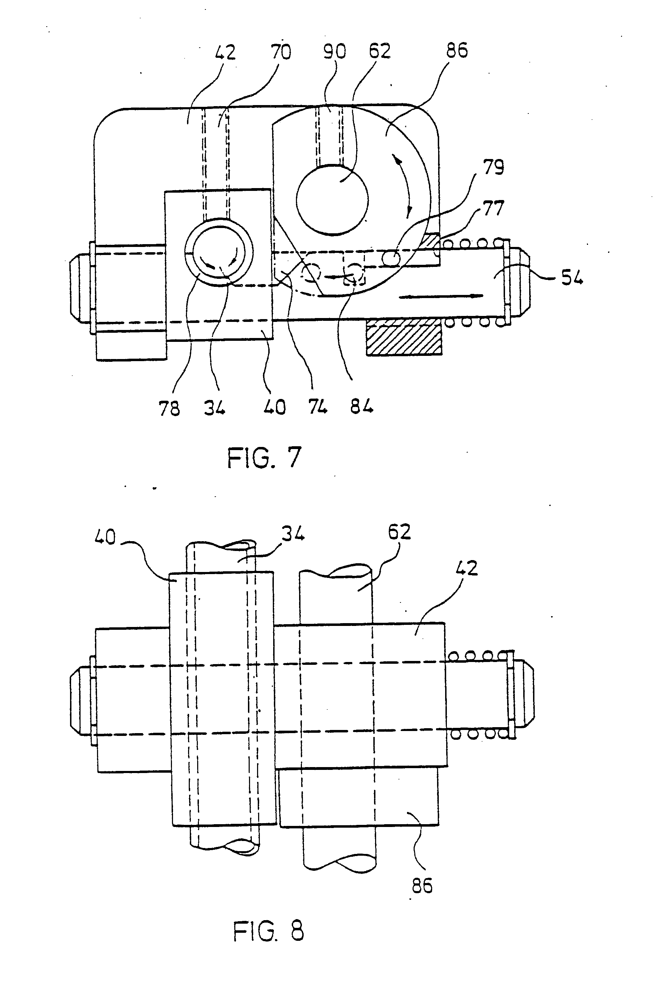

Fig. 7 eine vergrößerte Darstellung, teilweise geschnitten, des Lagerbockes mit dem Ausrückglied und dem Steuerelement, und

Fig. 8 eine Ansicht von unten des in Fig. 7 dargestellten Lagerbockes mit Ausrückglied und Steuerelement.

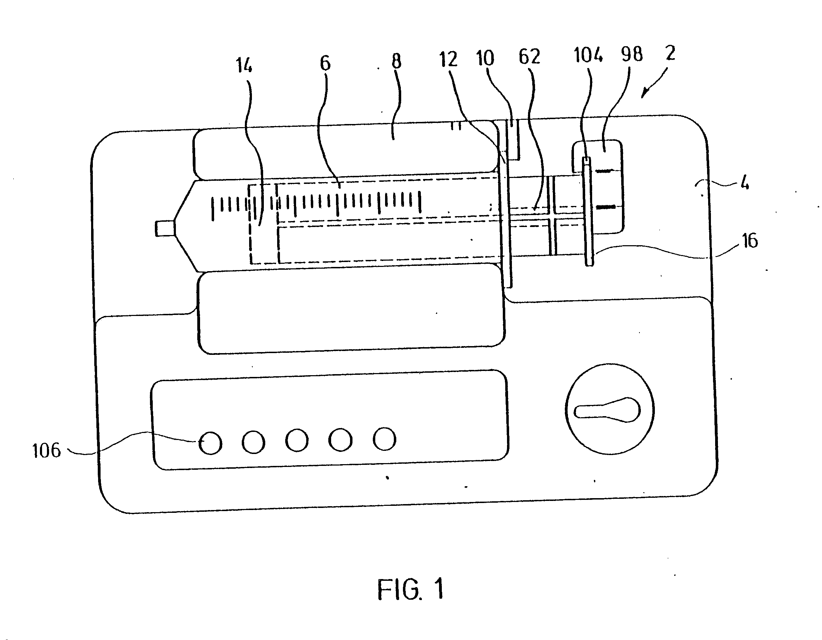

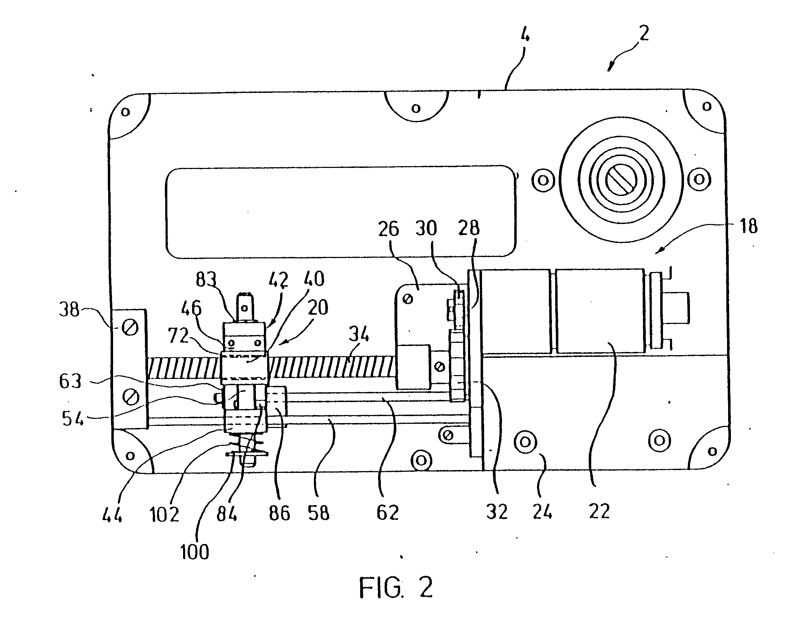

Die in den Fig. 1 und 2 dargestellte Spritzenpumpe 2 weist ein Gehäuse 4 auf, an dessen Oberseite eine den Spritzenkörper 6 am Umfang teilweise umfassende Halterung 8 vorgesehen ist. Mittels einer zwischen einem Halteteil 10 und der Halterung 8 festgelegten Spritzenschulter 12 ist die Spritze in Längsrichtung in der Halterung 8 festgelegt.

Im Inneren des Gehäuses 4 ist, wie aus Fig. 2 ersichtlich, ein Antrieb 18 und ein mit diesem in Verbindung stehendes Getriebe 20 untergebracht. Als Antrieb ist hier ein Elektromotor 22 vorgesehen, der an einem an einer Konsole 24 angebrachten Lagerbock 26 angeflanscht ist. Die Motorwelle 28 trägt ein Ritzel 30, welches mit einem Zahnrad 32 kämmt, das drehfest auf einer Gewindespindel 34 angeordnet ist.

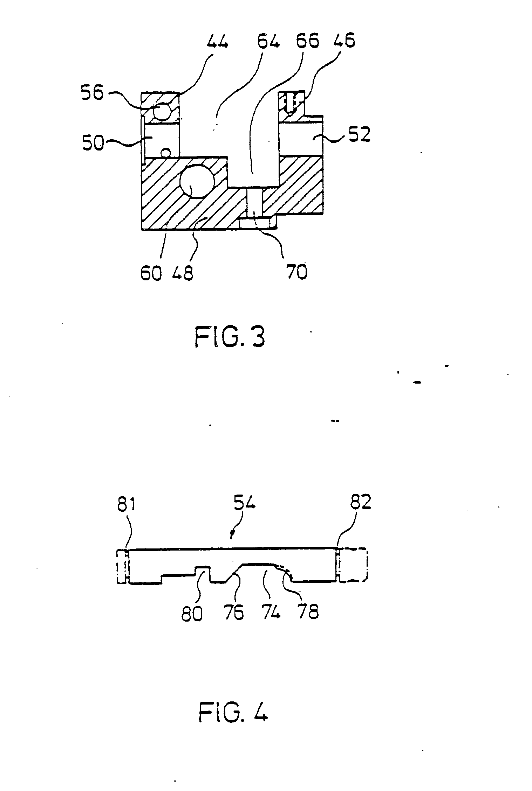

Die Gewindespindel 34 stellt einen Teil des Getriebes 20 dar, welches die Antriebsbewegung des Antriebs 18 auf den Spritzenkolben 14 trägt. Die Gewindespindel 34 ist parallel zur Motorwelle 28 ausgerichtet, wobei sie mit einem Ende im Lagerbock 26 drehbar gelagert ist, während das andere Ende von einem an der gegenüberliegenden Gehäusewand befestigten Lagerbock 38 drehbar und in axialer Richtung unverschiebbar gehalten ist. Weiterer Bestandteil des Getriebes 36 ist ein auf der Gewindespindel 34 angeordnetes längliches Führungsstück 40. Zur Halterung des Führungsstückes 40 ist ein Lagerbock 42 vorgesehen, welcher als Einzelteil in Fig. 3 dargestellt ist.

Der Lagerbock 42 ist im wesentlichen U-förmig ausgebildet und weist zwei Schenkel 44, 46 und einen Basisteil 48 auf. In den beiden Schenkeln 44, 46 des Führungsstückes 42 sind zueinander ausgerichtete Bohrungen 50, 52 vorgesehen, in denen ein als Kupplungsbolzen ausgebildetes Ausrückglied 54 angeordnet ist. Quer zu den Bohrungen 50, 52 ist im Schenkel 44 eine weitere Bohrung 56 vorgesehen, die eine parallel zur Gewindespindel 34 angeordnete, gehäusefest in den Lagerböcken 26, 28 angeordnete Verdrehsicherungsstange 58 aufnimmt. Eine parallel zur Bohrung 56 im Basisteil 48 des Lagerbockes 42 vorgesehene Bohrung 60 nimmt das Ende des als Antriebsstange ausgebildeten, parallel zur Verdrehsicherungsstange 58 ausgerichteten Antriebselementes 62 drehbar auf. Das Antriebselement 62 ist in axialer Richtung mittels eines Sicherungsringes 63, der in einer Ringnut des Antriebselementes 62 sitzt, gehalten. In einer U-förmigen Ausnehmung 64 des Lagerbockes 42 ist eine Vertiefung 66 ausgebildet, in der das Führungsstück 40 angeordnet und befestigt ist. Zur Befestigung des Führungsstückes 40 ist dabei im Basisteil 48 eine durchgehende Bohrung 70 vorgesehen, durch die eine (hier nicht dargestellte) Schraube geführt werden kann, die in ein an der Unterseite des Führungsstückes 40 vorgesehenes Gewinde eingreift. Das Führungsstück 40 weist eine parallel zu den Bohrungen 56, 60 verlaufende Bohrung 72 auf (in Fig. 2 gestrichelt angedeutet), durch die die Gewindespindel 34 mit Gleitsitz geführt ist. Die Gewindespindel 34 und das Führungsstück 40 bilden somit eine verkippfreie Führung für den Lagerbock 42 und das Ausrückglied 54. Eine Verdrehung der Anordnung wird durch die Verdrehsicherungsstange 58 verhindert, die in der Bohrung 56 mit Spiel geführt ist.

Das in Fig. 4 als Einzelteil dargestellte, als Kupplungsbolzen ausgebildete Ausrückglied 54 weist eine quer zu seiner Längsachse verlaufende Ausnehmung 74 auf, die axial auf einer Seite durch eine schräg zur Achse verlaufende Wand 76 und auf der anderen Seite durch ein Gewindesegment 78 begrenzt ist. In dieser Ausnehmung 74 ist die Gewindespindel 34 mit einem Teil ihres Umfanges angeordnet, so daß das Gewinde der Gewindespindel 34 mit dem Gewindesegment 78 in Eingriff bringbar ist. Die axiale Länge der Ausnehmung 74 ist so gewählt, daß bei einer axialen Verschiebung des Ausrückgliedes 54 das Gewinde der Gewindespindel 34 vollständig außer Eingriff mit dem Gewindesegment 78 kommt, bevor das Ausrückglied 54 mit einer Schulter 77 an einem Stift 79 zur Anlage kommt. Neben der Ausnehmung 74 ist eine weitere Ausnehmung 80 im Ausrückglied 54 vorgesehen. In diese Ausnehmung 80 greift ein sich axial erstreckender Stift 84 ein, der fest mit einem Steuerelement 86 verbunden ist, welches drehfest auf dem Antriebselement 62 sitzt. An den Enden weist das Ausrückglied 54 Ringnuten 81, 82 auf, in deren eine ein Sicherungsring 83 eingreift, der die Verschiebelänge des Ausrückgliedes 54 begrenzt.

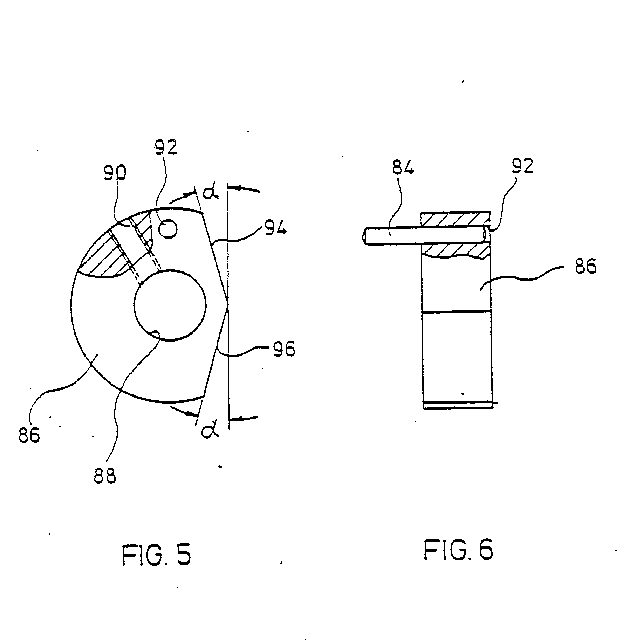

Das Steuerelement 86 ist als Einzelteil in den Fig. 5 und 6 dargestellt. Es besteht aus einem Kreissegment, welches eine zentrale Bohrung 88 aufweist, durch die das Antriebselement 62 geführt ist. Zur drehfesten Verbindung des Steuerelementes 86 mit dem Antriebselement 62 ist im Steuerelement 86 eine radiale Gewindebohrung 90 vorgesehen, in die eine (nicht dargestellte) Schraube einschraubbar ist, mit der das Steuerelement 86 auf dem Antriebselement 62 festgeklemmt wird. Exzentrisch zur Bohrung 88 ist eine weitere Bohrung 92 vorgesehen, die den Stift 84 aufnimmt. Der Stift 84 sitzt beispielsweise mit Preßsitz fest in der Bohrung 92 und erstreckt sich parallel zur Achse der Bohrung 88. Um eine kompakte Anordnung zu ermöglichen ist das scheibenförmige Steuerelement 84 segmentartig ausgebildet, wobei es sich neben der Bohrung 88 schneidende ebene Flächen 94, 96 bildet. Die Flächen 94, 96 verlaufen bezüglich einer Ebene, die parallel zu der die Achsen der Bohrungen 88, 92 enthaltenden Ebene verläuft, unter einem spitzen Winkel $\alpha$ von insbesondere 15°.

Nachfolgend ist die Arbeitsweise der Spritzenpumpe 2 näher beschrieben. Um die Spritzenpumpe 2 betriebsbereit zu machen, wird eine mit einer Flüssigkeit gefüllte Spritze in der Halterung 8 befestigt. Der Spritzenkolben 14 ist in diesem Zustand relativ weit aus dem Spritzenkörper 6 herausgezogen. Um das mit dem Antriebselement 62 fest verbundene Anschlußstück 98 in Eingriff mit der Druckplatte 16 des Spritzenkolbens 14 zu bringen, muß das Antriebselement 62 entsprechend weit herausgezogen werden. Dazu ist es zunächst erforderlich, das Antriebselement 62 mittels des Ausrückgliedes 54 vom Antrieb 18 zu trennen. Das Antriebselement 62 wird dabei mittels des Anschlußstückes 98 um seine Längsachse gedreht. Das fest mit dem Antriebselement 62 verbundene Steuerelement 86 wird dabei gleichfalls um die Längsachse gedreht, so daß der damit verbundene Stift 84 entlang eines Kreisbogenabschnittes um diese Achse verschwenkt wird. Dabei wird die Drehbewegung in eine Translationsbewegung des Ausrückgliedes 54 umgewandelt, in dessen Ausnehmung 80 der Stift 84 eingreift. Das Ausrückglied 54 wird dabei entgegen einer konzentrisch um das aus dem Lagerbock 42 herausragende Ende des Ausrückgliedes 54 angeordneten, einerseits am Lagerbock 42 und andererseits an einem mit dem Ausrückglied 54 verbundenen, in der Ringnut 81 sitzenden Federteller 100 abgestützten Feder 102 verschoben, so daß das Gewindesegment 78 des Ausrückgliedes 54 außer Eingriff mit dem Gewinde der Gewindespindel 34 kommt. Das Antriebselement 62 kann dann entsprechend weit herausgezogen werden, so daß ein am Anschlußstück 98 vorgesehener radialer Schlitz 104 in Eingriff mit der Druckplatte 16 gebracht werden kann. Dazu braucht nach einer entsprechenden axialen Ausrichtung lediglich das Anschlußstück 98 losgelassen zu werden. Infolge der Kraft der Feder 102 wird dadurch das Ausrückglied 54 wieder in Eingriff mit dem Gewinde gebracht, und das Steuerelement 82 und damit das Antriebselement 62 werden in ihre Ausgangsstellung zurückbewegt, in der der Schlitz 104 am Anschlußstück 98 die Druckplatte 16 am Rande umgreift.

Die Betätigung der Spritzenpumpe 2 erfolgt dann durch Einschalten des Elektromotors 22, mittels eines an der Oberseite des Gehäuses 4 vorgesehenen Schalters 106. Infolge der Drehung der Motorwelle 28 des Elektromotors 22 wird über das Ritzel 30 und das Zahnrad 32 die Gewindespindel 34 angetrieben, so daß sich der Lagerbock 42 und das Führungsstück 40 entlang der Gewindespindel 34 bewegen. Da das Antriebselement 62 in dem Lagerbock 42 axial festgelegt ist, wird es mitgenommen und bewegt dadurch den Spritzenkolben 14 in den Spritzenkörper hinein. Nach dem Ausschalten des Elektromotors 22 kann die Spritze, wie zuvor beschrieben, auf einfache Weise wieder in den Ausgangszustand versetzt werden.

**Patentansprüche**

1. Spritzenpumpe zur dosierenden Förderung einer Flüssigkeit aus einem Spritzenzylinder (6) mittels eines Spritzenkolbens (14) wenigstens einer auswechselbaren Spritze, mit einem Gehäuse (4), das eine Halterung (8) für die Spritze aufweist, mit einem motorischen Antrieb, mit einem Schraubspindelgetriebe (20) zur Übertragung der motorischen Antriebskraft vom Antrieb auf die Spritze, wobei das Schraubspindelgetriebe (20) ein axial verschiebbares Antriebselement (62) aufweist, dessen eines Ende mit dem beweglichen Spritzenteil außerhalb des Gehäuses in Eingriff bringbar ist, um das bewegliche Spritzenteil translatorisch zu bewegen und dessen anderes Ende mit einer in einem Lagerbock (42) angeordneten translatorisch bewegbaren Kupplung, die ein mit einem Gewindesegment (78) versehenes Ausrückglied (54) aufweist, um eine Antriebsverbindung herzustellen und zu trennen verbunden ist, wobei der Lagerbock ein längliches Führungsstück (40) trägt, das im Gleitsitz auf der Gewindespindel (34) angeordnet ist, und wobei das Ausrückglied der Kupplung am Führungsstück und/oder am Lagerbock verschiebbar gelagert ist, und wobei ferner das Ausrückglied ein quer zur Gewindespindel verschiebbar gelagerter Kupplungsbolzen ist, der eine sich quer zu seiner Längsachse erstreckende, im wesentlichen U-förmige Ausnehmung (74) aufweist, durch die sich die Gewindespindel mit zumindest einem Teil ihres Umfanges erstreckt, und an deren einer Flanke der U-förmigen Ausnehmung das Gewindesegment ausgebildet ist, dadurch gekennzeichnet, daß die Gewindespindel (34) innerhalb des Gehäuses (4) angeordnet und gelagert ist, daß sich das Antriebselement (62) durch die Gehäusewandung hindurch erstreckt, wobei das andere Ende des Antriebselementes (62) innerhalb des Gehäuses (4) am Ausrückglied (54) angelenkt ist und daß das Antriebselement (62) zur Betätigung der Kupplung um seine Längsachse drehbar im Gehäuse (4) gelagert ist.

2. Spritzenpumpe nach Anspruch 1, dadurch gekennzeichnet, daß das Antriebselement (62) ein separates als Antriebsstange ausgebildetes Bauteil ist, das im Abstand zur Gewindestange (34) im Gehäuse (4) angeordnet ist.

3. Spritzenpumpe nach Anspruch 1 oder 2, dadurch gekennzeichnet, daß das außerhalb des Gehäuses (4) angeordnete Ende des Antriebselementes (62) mit einem Anschlußstück (98) versehen ist.

4. Spritzenpumpe nach einem der vorhergehenden Ansprüche, dadurch gekennzeichnet, daß der Lagerbock (42) axial verschiebbar auf einer gehäusefesten Verdrehsicherungsstange (58) angeordnet ist.

5. Spritzenpumpe nach einem der vorhergehenen Ansprüche, dadurch gekennzeichnet, daß der Lagerbock (42) im wesentlichen U-förmig ausgebildet ist, wobei einer der die U-Form bildenden Schenkel (44, 46) des Lagerbocks (42) eine Bohrung (56) zur Aufnahme einer Verdrehsicherungsstange (58) aufweist.

6. Spritzenpumpe nach Anspruch 5, dadurch gekennzeichnet, daß beide Schenkel (44, 46) des Lagerbocks (42) Bohrungen (50, 52) aufweisen, in denen das Ausrückglied (54) axial verschiebbar gelagert ist.

7. Spritzenpumpe nach Anspruch 5 oder 6, dadurch gekennzeichnet, daß der Lagerbock (42) eine Bohrung (60) aufweist, in der das mit dem bewegbaren Spritzenteil in Eingriff bringbare Antriebselement (62) drehbar und axial unverschiebbar gelagert ist.

8. Spritzenpumpe nach einem der Ansprüche 5-7, dadurch gekennzeichnet, daß das Führungsstück (40) in einerdurch die Schenkel (44, 46) des Lagerbocks (42) gebildeten U-förmigen Ausnehmung (64) befestigt ist.

9. Spritzenpumpe nach einem der vorhergehenden Ansprüche, dadurch gekennzeichnet, daß zwischen dem mit dem beweglichen Spritzenteil in Eingriff bringbaren Antriebselement (62) und dem Ausrückglied (54) ein Steuerelement (82) angeordnet ist, um eine Drehbewegung des Antriebselements (62) in eine translatorische Bewegung des Ausrückgliedes (54) umzusetzen.

10. Spritzenpumpe nach Anspruch 9, dadurch gekennzeichnet, daß das Steuerelement (82) eine drehbar gelagerte Scheibe ist, die mit dem Antriebselement in Eingriff ist und die einen Mitnehmer (84) aufweist, der mit dem Ausrückglied (54) in Eingriff ist.

11. Spritzenpumpe nach Anspruch 10, dadurch gekennzeichnet, daß die Scheibe am Antriebselement (62) befestigt ist.

12. Spritzenpumpe nach Anspruch 10 oder 11, dadurch gekennzeichnet, daß der Mitnehmer (84) ein exzentrisch zur Drehachse der Scheibe angeordneter Stift ist, der in eine Ausnehmung des Ausrückgliedes (54) eingreift.

**Claims**

1. Spray pump for metered delivery of a liquid from a spray cylinder (6) by means of a spray piston (14) with at least one interchangeable spray, with a housing (4) which has a mounting (8) for the spray, with a motor drive having a screwed-shaft gear unit (20) to transmit the driving power of the motor from the drive to the spray, the screwed-shaft gear unit (20) having an axially movable drive element (62) one end of which can be made to engage the movable spray part outside the housing to move the movable spray part translationally and whose other end is connected to a translationally movable coupling arranged in a bearing block (42), said coupling being provided with a stop link (54) provided with a threaded section (78), to make and break a drive connection, the bearing block supporting an elongated guide piece (40) arranged in the sliding seat on the threaded shaft (34), and the stop link of the coupling being supported movably on the guide piece and/or on the bearing block, the stop link furthermore being a coupling pin supported movably at right angles to the threaded shaft and having a recess (74) extending at right angles to its longitudinal axis and essentially U-shaped, through which at least part of the circumference of the threaded shaft extends, and on one side of the U-shaped recess of which the threaded section is provided, characterized in that the threaded shaft (34) is arranged and supported inside the housing (4), that the drive element (62) extends through the housing wall, the other end of the drive element (62) pivoting on stop link (54) inside the housing and that the drive element (62) pivots around its longitudinal axis in the housing (4) to operate the coupling.

2. Spray pump according to claim 1, characterized in that the drive element (62) is a separate component designed as a drive rod and is located at a distance from the threaded rod (34) in the housing (4).

3. Spray pump according to claim 1 or 2, characterized in that the end of the drive element (62) located outside the housing (4) is equipped with a connecting piece (98).

4. Spray pump according to one of the preceding claims, characterized in that the bearing block (42) is arranged to be movable axially on a torsional locking rod (58) fixed to the housing.

5. Spray pump according to one of the preceding claims, characterized in that the bearing block (42) is of essentially U-shaped design, one of the sides (44, 46) of the bearing block (42) forming the U-shape having a drill hole (56) to receive a torsional locking rod (58).

6. Spray pump according to claim 5, characterized in that both sides (44, 46) of the bearing block (42) have drill holes (50, 52) in which the stop link (54) is supported and axially movable.

7. Spray pump according to claim 5 or 6, characterized in that the bearing block (42) has a drill hole (60) in which the drive element (62) which can engage the movable spray part, pivots and is axially immovable.

8. Spray pump according to one of claims 5-7, characterized in that the guide piece (40) is secured in a U-shaped recess (64) formed by the sides (44, 46) of the bearing block (42).

9. Spray pump according to one of the preceding claims, characterized in that a control element (82) is located between the drive element (62) able to engage the movable spray part and the stop link (54) in order to convert the rotation of the drive element (62) into a translational movement of the stop link (54).

10. Spray pump according to claim 9, characterized in that control element (82) is a pivoting disk engaging the drive element and having a dog (84) which engages the stop link (54).

11. Spray pump according to claim 10, characterized in that the disk is attached to the drive element (62).

12. Spray pump according to claim 10 or 11, characterized in that the dog (84) is a pin arranged eccentrically to the axis of rotation of the disk and engaging a recess in the stop link (54).

**Revendications**

1. Pompe d'injection destinée à expulser un liquide d'un cylindre de seringue (6) en une quantité dosée au moyen d'un piston de seringue (14) d'au moins une seringue interchangeable, cette pompe comprenant un boîtier (4) qui présente une monture (8) destinée à porter la seringue, un entraînement motorisé comportant un mécanisme à vis (20) pour transmettre la force de l'entraînement motorisé, de cet entraînement à la seringue, le mécanisme à vis (20) comprenant un élément d'entraînement (62) mobile en translation axiale, dont une extrémité peut être mise en prise avec la partie mobile de la seringue située en dehors du boîtier, et dont l'autre extrémité est reliée à un embrayage mobile en translation disposé dans un support palier (42), qui présente un organe de débrayage (52) muni d'un segment de filetage (78), pour établir et supprimer la liaison cinématique, le support palier portant une pièce de guidage (40) de forme allongée qui est montée à glissement doux sur la vis (34), l'organe de débrayage de l'embrayage étant monté mobile en translation dans la piève de guidage et/ou sur le support palier, et l'organe de débrayage étant constitué par une broche d'embrayage qui est montée mobile en translation par rapport à la vis et qui présente un évidement (74) sensiblement en forme de U, qui s'étend transversalement à son axe longitudinal, et à travers lequel la vis est engagée par au moins une partie de sa circonférence, et le segment de filetage étant formé sur un des flancs de l'évidement en U, caractérisée en ce que la vis (34) est disposée et tourillonnée à l'intérieur du boîtier (4), en ce que l'élément d'entraînement (62) s'étend à travers la paroi du boîtier, l'autre extrémité de l'élément d'entraînement (62) étant articulée sur l'organe de débrayage (54) à l'intérieur du boîtier (4), et en ce que l'élément d'entraînement (62) est monté dans le boîtier (4) de façon à pouvoir tourner autour de son axe longitudinal, pour actionner l'embrayage.

2. Pompe d'injection selon la revendication 1, caractérisée en ce que l'élément d'entraînement (62) est un élément séparé, constitué par une tige d'entraînement qui est disposée dans le boîtier (4) à distance de la vis (34).

3. Pompe d'injection selon la revendication 1 ou 2, caractérisée en ce que l'extrémité de l'élément d'entraînement (62) qui est disposée à l'extérieur du boîtier (4) est munie d'une pièce de raccordement (98).

4. Pompe d'injection selon une des revendications précédentes, caractérisée en ce que le support palier (42) est disposé mobile en translation axiale sur une tige (58) de blocage en rotation solidaire du boîtier.

5. Pompe d'injection selon une des revendications précédentes, caractérisée en ce que le support palier (42) est d'une configuration sensiblement en U, l'une des branches (44, 46) du support palier (42) qui forment la configuration en U présentant un perçage (56) destiné à recevoir une tige de blocage en rotation (58).

6. Pompe d'injection selon la revendication 5, caractérisée en ce que les deux branches (44, 46) du support palier (42) présentent des perçages (50, 52) dans lesquels l'organe de débrayage (54) est monté mobile en translation axiale.

7. Pompe d'injection selon la revendication 5 ou la revendication 6, caractérisée en ce que le support palier (42) présente un perçage (60) dans lequel l'élément d'entraînement (62) qui peut être mis en prise avec la partie mobile de la seringue est monté libre en rotation mais bloqué en translation axiale.

8. Pompe d'injection selon une des revendications 5 à 7, caractérisée en ce que la pièce de guidage (40) est fixée dans un évidement en U (64) formé par les branches (44, 46) du support palier (42).

9. Pompe d'injection selon une des revendications précédentes, caractérisée en ce que, entre l'élément d'entraînement (62) qui peut être mis en prise avec la partie mobile de la seringue et l'organe de débrayage (54), est disposé un élément de commande (82), servant à transformer un mouvement de rotation de l'élément d'entraînement (62) en un mouvement de translation de l'organe de débrayage (54).

10. Pompe d'injection selon la revendication 9, caractérisée en ce que l'élément de commande (82) est un disque monté tournant qui est en prise avec l'élément d'entraînement et qui présente un entraîneur (84) qui est en prise avec l'organe de débrayage (54).

11. Pompe d'injection selon la revendication 10, caractérisée en ce que le disque est fixé à l'élément d'entraînement (62).

12. Pompe d'injection selon la revendication 10 ou la revendication 11, caractérisée en ce que l'entraîneur (84) est un doigt disposé excentriquement par rapport à l'axe de rotation du disque et qui est engagé dans un évidement de l'organe de débrayage (54).

0 190 269

FIG. 1

FIG. 2

0 190 269

56 — 44 — 64 — 66 — 46

50 —

52

60 — 48 — 70

# FIG. 3

81 — 54 — 82

80 — 76 — 74 — 78

# FIG. 4

FIG. 5

FIG. 6

FIG. 7

FIG. 8